# EUROPEAN PATENT APPLICATION

(11) **EP 4 483 941 A1**
(43) Date of publication of application: **01.01.2025**
(21) Application number: 24184966.0
(22) Date of filing: 27.06.2024
(51) Int. Cl.: A61N 1/05, A61N 1/36, A61N 1/375

(54) **COCHLEAR IMPLANT ELECTRODE ARRAYS AND ELECTRODE ARRAY COVER ASSEMBLIES FOR USE WITH THE SAME**

(30) Priority: 30.06.2023 US 202363511622 P
(71) Applicant: Advanced Bionics LLC, Valencia, CA 91355 (US)
(72) Inventor: Gulati, Soumya, Stevenson Ranch, CA 91381 (US); Salvatierra, Candelaria, Lancaster, CA 91356 (US); Patnala, Anil, Stevenson Ranch, CA 91381 (US); Spahr, Anthony J., Oxnard, CA 93035 (US)
(74) Representative: Schwan Schorer & Partner mbB

(57) **Abstract**

An apparatus in accordance with at least one of the present inventions comprises a cochlear implant including an electrode array with an array body that defines an outer surface and a plurality of electrically conductive contacts on the array body, and an electrode array cover assembly including an electrode array cover including a wall, defining an inner surface and an outer surface, and a plurality of spaced cover apertures that extend through an apertured portion of the wall from the inner surface to the outer surface and that are at least substantially aligned with the electrically conductive contacts, and means, at least partially within the electrode array cover, for holding the array body straight and against the inner surface of the apertured portion of the wall in such a manner that a fluidic seal is formed between the array body and the apertured portion of the wall.

## Description

### BACKGROUND

### 1. Field

The present disclosure relates generally to the implantable portion of implantable cochlear stimulation (or "ICS") systems and, in particular, to electrode arrays.

### 2. Description of the Related Art

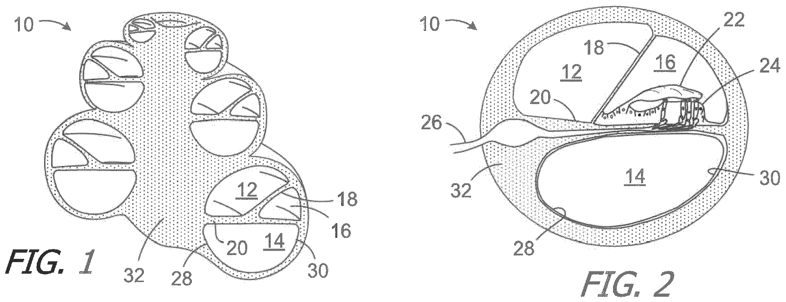

Referring to FIGS. 1 and 2, the cochlea 10 is a hollow, helically coiled, tubular bone (similar to a nautilus shell) that is divided into the scala vestibuli 12, the scala tympani 14 and the scala media 16 by the Reissner's membrane 18 and the basilar membrane 20. The cochlea 10, which typically includes approximately two and a half helical turns, is filled with a fluid that moves in response to the vibrations coming from the middle ear. As the fluid moves, a tectorial membrane 22 and thousands of hair cells 24 are set in motion. The hair cells 24 convert that motion to electrical signals that are communicated via neurotransmitters to the auditory nerve 26, and transformed into electrical impulses known as action potentials, which are propagated to structures in the brainstem for further processing. Many profoundly deaf people have sensorineural hearing loss that can arise from the absence or the destruction of the hair cells 24 in the cochlea 10. Other aspects of the cochlea 10 illustrated in FIGS. 1 and 2 include the medial wall 28, the lateral wall 30 and the modiolus 32.

ICS systems are used to help the profoundly deaf perceive a sensation of sound by directly exciting the intact auditory nerve with controlled impulses of electrical current. Ambient sound pressure waves are picked up by an externally worn microphone and converted to electrical signals. The electrical signals, in turn, are processed by a sound processor, converted to a pulse sequence having varying pulse widths, rates, and/or amplitudes, and transmitted to an implanted receiver circuit of the ICS system. The implanted receiver circuit is connected to an implantable lead with an electrode array that is inserted into the cochlea of the inner ear, and electrical stimulation current is applied to varying electrode combinations to create a perception of sound. The electrode array may, alternatively, be directly inserted into the cochlear nerve without residing in the cochlea. A representative ICS system is disclosed in U.S. Patent No. 5,824,022, which is entitled "Cochlear Stimulation System Employing Behind-The-Ear Sound processor With Remote Control" and incorporated herein by reference in its entirety. Examples of commercially available ICS sound processors include, but are not limited to, the Advanced Bionics^{™} Harmony^{™} BTE sound processor, the Advanced Bionics^{™} Naida^{™} BTE sound processor and the Advanced Bionics^{™} Neptune^{™} body worn sound processor.

As alluded to above, some ICS systems include an implantable cochlear stimulator (or "cochlear implant") having a lead with an electrode array, a sound processor unit (e.g., a body worn processor or behind-the-ear processor) that communicates with the cochlear implant, and a microphone that is part of, or is in communication with, the sound processor unit. The cochlear implant electrode array includes a flexible body formed from a resilient material and a plurality of electrically conductive contacts (e.g., sixteen platinum contacts) spaced along a surface of the flexible body. The cross-sectional size of the flexible body may taper from the base (or "basal") end to the tip (or "apical") end. The contacts of the array are connected to lead wires that extend through the flexible body. Exemplary cochlear leads are illustrated in WO2018/031025A1 and WO2018/102695A1, which are incorporated herein by reference in their entireties.

Some electrode arrays have a flexible body with a pre-curved shape, which causes the array to conform to the shape of the cochlea, and an embedded shape memory polymer (SMP) element or shape memory alloy (SMA) element (collectively "shape memory element") that maintains the flexible body in a straight (but flexible) state until implantation is complete. It is intended that the shape memory element will soften, and allow the flexible body to return to the pre-curved shape, in response to a combination of an increase in temperature, typically to body temperature, and the presence of moisture post implantation. Alternatively, or in addition, some electrode arrays are drug eluting arrays that include one or more drugs which may be doped into the flexible body or applied to the exterior of the flexible body. One exemplary drug is the antifibrotic drug dexamethasone (DEX), which is activated by moisture.

The present inventors have determined that electrode arrays with shape memory elements should be maintained in a straight state during shipping and storage, and that drugs which are embedded into the flexible body, or applied to the surface thereof, should be kept dry until the electrode array is inserted into the cochlea. As used herein, an electrode array that is "maintained in straight (or straightened) state" is either maintained with no curvature over its length or, in those instances where the unstressed molded shape of the electrode array is such that there is slight curvature (e.g., a slight curvature at the tip end), is maintained either with no curvature over its length or in the unstressed molded shape with the slight curvature. The present inventors have further determined that conventional methods of packaging electrode arrays are susceptible to improvement. For example, electrode arrays are frequently packaged in a hollow cylindrical tube. The present inventors have determined that the tapering of the flexible body results in the electrode array being unconstrained in all three rotation degrees of freedom and all three translational degrees of freedom at the tip end despite a tight fit at the base end, which can result in premature tip curling. Premature tip curling may result in tip fold-over within the cochlea and/or trauma to the delicate structures inside the cochlea. In some instances, premature tip curling can make the insertion process more challenging or even impossible. Accordingly, it is important to keep the shape memory electrode fully constrained during shipping and storage to prevent shipping and storage conditions from affecting the shape memory element. Another issue relates to various pre-implantation procedures that involve the use of saline. The sterile tray, which includes all of the cochlear implant components, is soaked in saline to remove static buildup. Functional testing immediately prior to implantation also involves submerging the electrode array in saline so that all of the contacts are exposed to the saline for impedance testing. Such exposure to saline is, however, problematic when drugs are coated on or embedded into the flexible body or applied to the surface thereof.

### SUMMARY

An apparatus in accordance with at least one of the present inventions comprises a cochlear implant including an electrode array with an array body that defines an outer surface and a plurality of electrically conductive contacts on the array body, and an electrode array cover assembly including an electrode array cover including a wall, defining an inner surface and an outer surface, and a plurality of spaced cover apertures that extend through an apertured portion of the wall from the inner surface to the outer surface and that are at least substantially aligned with the electrically conductive contacts, and means, at least partially within the electrode array cover, for holding the array body straight and against the inner surface of the apertured portion of the wall in such a manner that a fluidic seal is formed between the array body and the apertured portion of the wall.

An apparatus in accordance with at least one of the present inventions comprises a cochlear lead including an electrode array with an array body that defines an outer surface and a plurality of electrically conductive contacts on the array body, and an electrode array cover assembly including an electrode array cover including a wall, defining an inner surface and an outer surface, and a plurality of spaced cover apertures that extend through an apertured portion of the wall from the inner surface to the outer surface and that are at least substantially aligned with the electrically conductive contacts, and a resilient insert at least partially within the electrode array cover and compressed between the inner surface of the electrode array cover and the outer surface of the electrode array, wherein the respective configurations of the array body and the resilient insert are such that the compressed resilient insert holds the array body straight and against the inner surface of the apertured portion of the wall in such a manner that a fluidic seal is formed between the array body and the apertured portion of the wall.

An apparatus in accordance with at least one of the present inventions comprises an electrode array cover including first and second separable wall members that are secured to one another, the first wall member defining an inner surface, an outer surface, and a plurality of spaced cover apertures that extend through an apertured portion of the first wall member from the inner surface to the outer surface, and the second wall member defining an inner surface and an outer surface, and a cochlear lead including an electrode array with an array body that defines an outer surface and a plurality of electrically conductive contacts on the array body that are at least substantially aligned with the cover apertures, wherein the respective configurations of the array body and the inner surface of the second wall member are such that the second wall member holds the array body against the inner surface of the apertured portion of the first wall member in such a manner that a fluidic seal is formed between the array body and the apertured portion of the first wall member.

An apparatus in accordance with at least one of the present inventions comprises a cochlear implant including an electrode array with an array body that defines an outer surface and a plurality of electrically conductive contacts on the array body, and a brace secured to the outer surface of the array body and configured to maintain the array body in straight state.

An apparatus in accordance with at least one of the present inventions comprises a cochlear implant including an electrode array with an array body that defines an outer surface and a plurality of electrically conductive contacts on the array body, and a case, in which at least the array body is located, that is configured to maintain the array body in straight state.

There are a number of advantages associated with such apparatus. By way of example, but not limitation, the present electrode array covers, braces and cases maintain the electrode array in a straightened state, thereby preventing premature curling. In at least some instances, the covers also create a seal that allows the contacts to be exposed to liquid (e.g., saline during static removal and functional testing) while preventing the liquid from reaching other portions of the electrode array and aversely effecting a shape memory element and/or drugs. In at least some instances, the braces and case also create a seal that prevents liquid from reaching the drugs on the electrode array.

The above described and many other features of the present inventions will become apparent as the inventions become better understood by reference to the following detailed description when considered in conjunction with the accompanying drawings.

### BRIEF DESCRIPTION OF THE DRAWINGS

Detailed descriptions of the exemplary embodiments will be made with reference to the accompanying drawings.
FIG. 1 is a section view of a cochlea.
FIG. 2 is another section view of the cochlea.
FIG. 3 is a top view of an apparatus including a cochlear implant and an electrode array cover assembly in accordance with one embodiment of a present invention.
FIG. 4 is a bottom view of a portion of the electrode array illustrated in FIG. 3.
FIG. 5 is a section view taken along line 5-5 in FIG. 4.
FIG. 6 is a section view taken along line 6-6 in FIG. 4.
FIG. 7 is an exploded side view of the electrode array cover assembly illustrated in FIG. 3.
FIG. 8 is a bottom view of a portion of the electrode array cover assembly illustrated in FIG. 3.
FIG. 9 is a section view taken along line 9-9 in FIG. 8.
FIG. 10 is a side view showing the electrode array cover assembly and electrode array illustrated in FIG. 3 being combined.
FIG. 11 is a bottom view showing the electrode array cover assembly and the electrode array illustrated in FIG. 3 in a combined state.
FIG. 12 is a partial section view taken along line 12-12 in FIG. 11.
FIG. 13 is a section view taken along line 13-13 in FIG. 11.
FIG. 14 is a section view taken along line 14-14 in FIG. 11.
FIG. 15 is an exploded side view of an electrode array cover assembly in accordance with one embodiment of a present invention.
FIG. 16 is a partial section view of the electrode array cover assembly illustrated in FIG. 15 and the electrode array illustrated in FIG. 15 in a combined state.
FIG. 17 is a side exploded side view of an electrode array cover in accordance with one embodiment of a present invention.
FIG. 18 is a bottom view of the electrode array cover illustrated in FIG. 17.
FIG. 19 is a section view taken along line 19-19 in FIG. 18.
FIG. 20 is a section view taken along line 20-20 in FIG. 18.
FIG. 21 is a side partial section view of a cochlear implant and the electrode array cover illustrated in FIG. 17 being combined.
FIG. 22 is a side partial section view of an apparatus including a cochlear implant and the electrode array cover illustrated in FIG. 17 in a combined state.
FIG. 23 is a section view taken along line 23-23 in FIG. 22.
FIG. 24 is a section view taken along line 24-24 in FIG. 22.
FIG. 25 is a side view of an electrode array brace in accordance with one embodiment of a present invention on an electrode array.
FIG. 26 is a perspective view of the electrode array brace and electrode array illustrated in FIG. 25.
FIG. 27 is an exploded perspective view of a portion of the electrode array brace and electrode array illustrated in FIG. 25.
FIG. 28 is an exploded perspective view of a portion of the electrode array brace and electrode array illustrated in FIG. 25.
FIG. 29 is a perspective view of the electrode array brace and electrode array illustrated in FIG. 25 with electrode array brace partially removed from the electrode array.
FIG. 30 is a perspective view of the electrode array brace and electrode array illustrated in FIG. 25.
FIG. 30A is an exploded section view of an electrode array brace in accordance with one embodiment of a present invention on an electrode array.
FIG. 30B is a section view of the electrode array brace illustrated in FIG. 30A, in a pre-shrunk state, adjacent to an electrode array.
FIG. 31 is a perspective view of an electrode array brace in accordance with one embodiment of a present invention on an electrode array.
FIG. 32 is a section view taken along line 32-32 in FIG. 31.
FIG. 33 is a perspective view of the electrode array brace and electrode array illustrated in FIG. 31.
FIG. 34 is a perspective view of an electrode array case in accordance with one embodiment of a present invention in an open state.
FIG. 35 is a perspective view of the electrode array case illustrated in FIG. 34 in an open state with an electrode array located within a portion of a storage volume.
FIG. 36 is a perspective view of the electrode array case illustrated in FIG. 34 in a closed state.
FIG. 37 is a perspective view of an electrode array case in accordance with one embodiment of a present invention in an open state with an electrode array located on a portion thereof.
FIG. 38 is a top view of the electrode array case and electrode array illustrated in FIG. 37.
FIG. 39 is a side view of a portion of the electrode array case illustrated in FIG. 37.
FIG. 40 is a side view of the electrode array case illustrated in FIG. 37 in a closed state with the electrode array located on a portion thereof.
FIG. 41 is a section view taken along line 41-41 in FIG. 40 with the electrode array omitted therefrom.
FIG. 42 is an exploded perspective view of a cochlear implant package in accordance with one embodiment of a present invention.
FIG. 43 is a top view of a portion of the cochlear implant package illustrated in FIG. 42.
FIG. 44 is a perspective view of a portion of the cochlear implant package illustrated in FIG. 42.
FIG. 45 is an exploded perspective view of a portion of the cochlear implant package illustrated in FIG. 42.
FIG. 46 is a top view of a portion of the cochlear implant package illustrated in FIG. 42.
FIG. 47 is a bottom view of a portion of the cochlear implant package illustrated in FIG. 42.

### DETAILED DESCRIPTION OF THE EXEMPLARY EMBODIMENTS

The following is a detailed description of the best presently known modes of carrying out the inventions. This description is not to be taken in a limiting sense, but is made merely for the purpose of illustrating the general principles of the inventions.

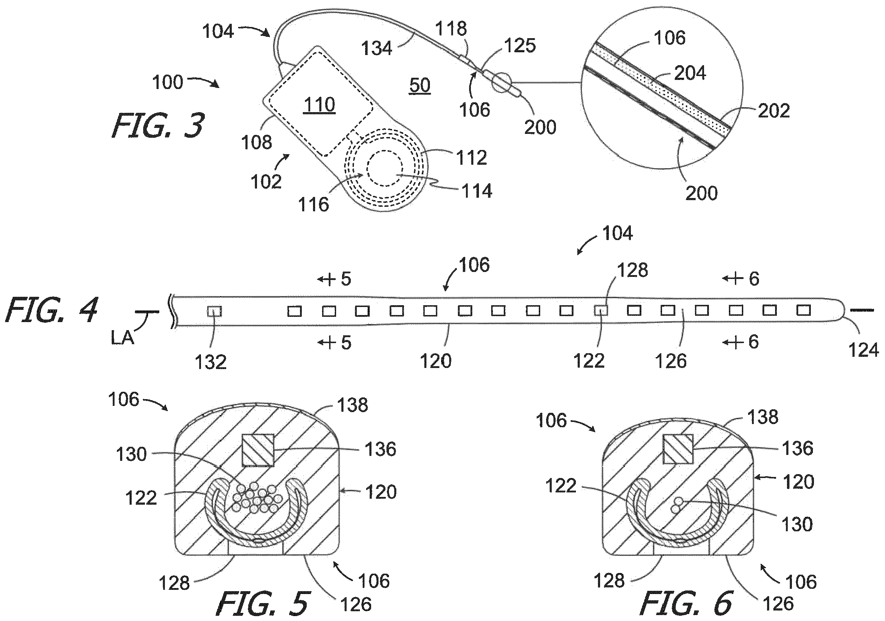

As illustrated for example in FIG. 3, an apparatus 50 in accordance with one embodiment of a present invention includes an implantable cochlear stimulator (or "cochlear implant") 100, with a stimulation assembly 102 and a cochlear lead 104 that has an electrode array 106, and an electrode array cover assembly (or "cover assembly") 200 on the electrode array. The exemplary cover assembly 200 includes a cover 202 and a resilient insert 204 that presses the electrode array 106 against the inner surface of the cover. The cover 202 and resilient insert 204 together maintain the electrode array 106 or other electrode array in a straightened state and also create a seal that allows the array contacts to be exposed to liquid (e.g., saline) while preventing the liquid from reaching other portions of the electrode array.

Referring first to the exemplary cochlear implant 100, the stimulation assembly 102 includes a flexible housing 108 formed from a silicone elastomer or other suitable material, a processor assembly 110, an antenna 112 that may be used to receive data and power by way of an external antenna that is associated with, for example, a sound processor unit, and a positioning magnet 114 located within a magnet pocket 116. The magnet 114 is used to maintain the position of a sound processor headpiece over the antenna 112. The cochlear implant may, in some instances, be configured in a manner that facilitates magnet removal and replacement. Here, the housing 108 may be provided with a magnet aperture (not shown) that extends from the magnet pocket 1 16 to the exterior of the housing.

In addition to the electrode array 106, the exemplary cochlear lead 104 illustrated in FIGS. 3-6 includes, in at least some instances, a wing 118 that functions as a handle for the surgeon during the implantation surgery. Other types of handles may also be employed. The exemplary electrode array 106 has a flexible body 120 and a plurality of electrically conductive contacts 122 (e.g., sixteen contacts) spaced along the flexible body between the tip end 124 of the flexible body and the base region 125 that is adjacent to the wing 118. The electrically conductive contacts 122 (or "contacts") may be located inward of the flexibly body outer surface 126 and exposed by way of a corresponding plurality of contact windows (or "windows") 128 that extend through the outer surface of the flexible body to the contacts. The contacts 122 are each connected to a respective lead wire 130 (FIGS. 5-6) that extends through the flexible body 120. In some instances, the flexible body 120 may include a depth (or "cochleostomy") marker 132 (FIG. 4) that is a predetermined distance from the tip end 124. In addition to functioning as a handle, the wing 118 provides tension relief for the lead wires 130 that do not run straight through the wing. A tubular member 134, which may consist of tubes of different sizes, extends from the wing 118 to the housing 108. The lead wires 130 extend through the tubular member to a connector (not shown) in the housing 108. The connection between the stimulation assembly 102 and the cochlear lead 104 may be a temporary connection, whereby the stimulation assembly and a cochlear lead may be disconnected from one another (e.g., for in situ replacement of the stimulation assembly), or a permanent connection.

Although the present inventions are not so limited, the flexible body 120 of the exemplary electrode array 106 illustrated in FIGS. 3-6 has a non-circular shape with a flat bottom in a cross-section perpendicular to the longitudinal axis LA, which defines the length direction of the electrode array. The flexible body 120 may also be tapered, with a perimeter in a plane perpendicular to the longitudinal axis LA that is smaller at the tip end 124 than at the base region 125. Any other suitable flexible body shape (e.g., circular or oval), with or without a flat surface, may also be employed. Suitable materials for the flexible body 120 include, but are not limited to, electrically non-conductive resilient materials such as liquid silicone rubber (LSR), high temperature vulcanization ("HTV") silicone rubbers, room temperature vulcanization ("RTV") silicone rubbers, and thermoplastic elastomers ("TPEs"). Suitable materials for the contacts 122 include, but are not limited to, platinum, platinum-iridium, gold and palladium. Although the present inventions are not limited to any particular electrode configuration, the exemplary contacts 122 may be generally U-shaped and may be formed by a placing a tubular workpiece into an appropriately shaped fixture, placing the end of a lead wire into the workpiece, and then applying heat and pressure to the workpiece to compress the workpiece onto the lead wire. The insulation may be removed from the portion of the lead wire within the workpiece prior to the application of heat and pressure or during the application of heat and pressure. Various examples of tubular workpieces being compressed onto lead wires are described in WO2018/031025A1 and WO2018/102695A1. The contact windows 128 extend from the outer surface 126 of the flexible body 120 to the contacts 122, thereby exposing portions of the contacts. In the exemplary implementation, the windows 130 are the same shape and expose the same portion of the associated contacts 122.

The exemplary electrode array 106 also has preset spiral shape (e.g. a helical shape) with a tight curvature (resulting from the mold shape) in an unstressed state that corresponds to the interior geometry of the cochlea. The spiral electrode array 106 may be maintained in a straightened prior until it is inserted into the cochlea with an embedded shape memory element 136 that is formed from a shape memory polymer (SMP) or shape memory alloy (SMA) element 136. The SMP element will soften, and allow the flexible body to return to the pre-curved shape, in response to an increase in temperature and the presence of moisture after implantation. Alternatively, in the case of an SMA element, it undergoes phase transition in response to stimuli like temperature that allows for a return to pre-curved shape. Suitable SMP materials for the shape memory element 136 include, but are not limited to, thiol-ene/acrylate-based polymers with shape memory properties, while suitable SMA materials include, but are not limited to, Nitinol. The electrode array 106 may also be a drug eluting electrode array that includes one or more drugs such as, for example, the antifibrotic drug dexamethasone (DEX), steroids and/or other elution capable active pharmaceutical ingredients (APIs). Here, a portion of the flexible body outer surface 126 may include a drug coating 138 which, in the illustrated embodiment is perimetrically offset from the contacts 122 and windows 128. The drug may also be doped into the flexible body material. DEX, which is activated by moisture, should be kept dry until the electrode array 106 is inserted into the cochlea.

Although the contacts 122 are all the same size and the windows 128 are all the same size in the illustrated embodiment, the contacts and/or windows may be different in sizes and/or shapes in other implementations. For example, the contacts may be larger in the portion of the array that will be positioned in the basal region of the cochlea than the contacts in the portion that will be positioned in the apical region of the cochlea. The position of the contacts may be such that a portion of each contact is aligned with the flexible body outer surface, thereby eliminating the need for a window.

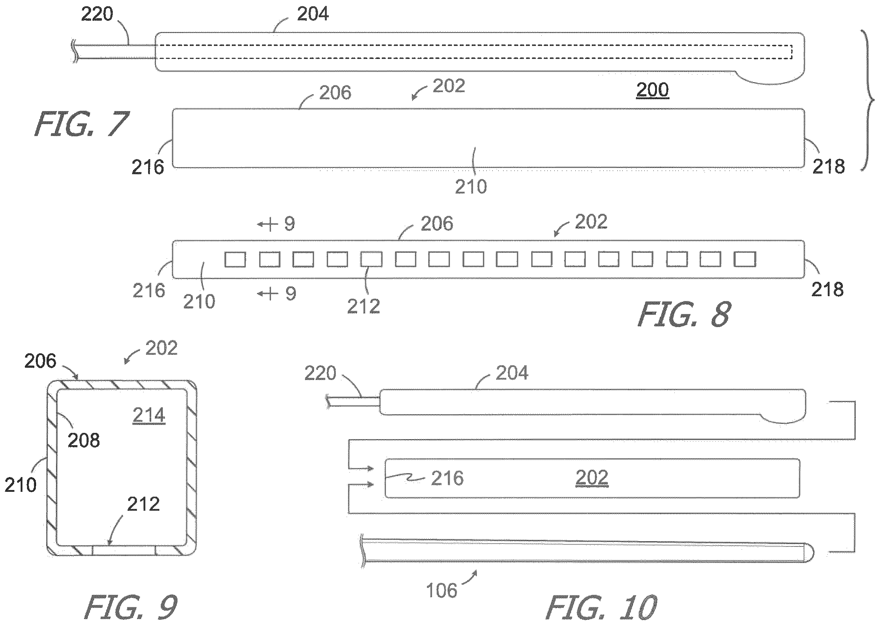

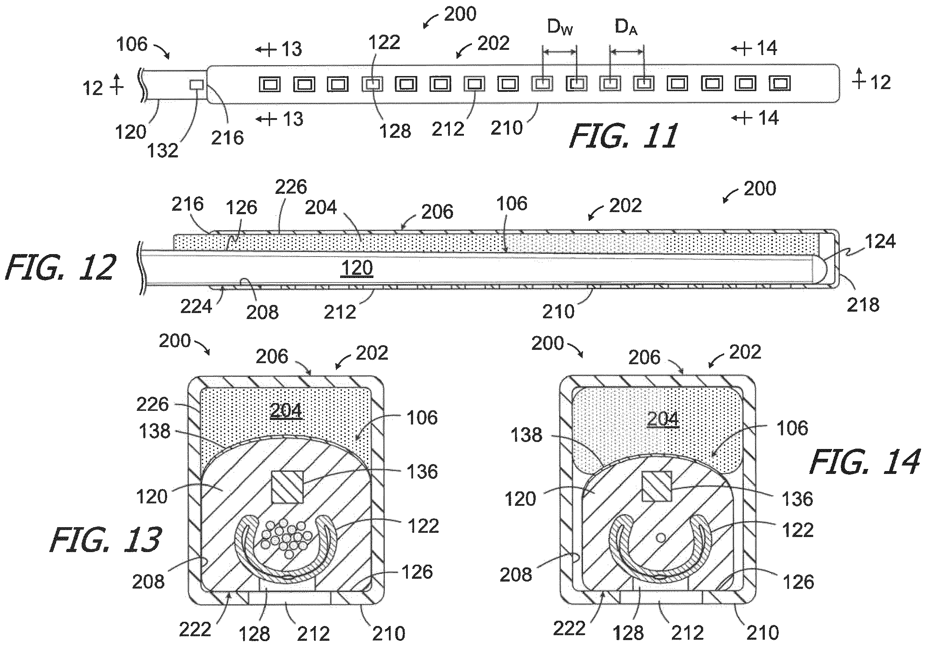

Turning to FIGS. 7-9, and as noted above, the exemplary electrode array cover assembly 200 includes a cover 202 and a resilient insert 204. The cover 202 includes a wall 206, with an inner surface 208 and an outer surface 210, and a plurality of spaced apertures 212 that extend through the wall from the inner surface the outer surface. The cover 202 also includes an interior volume 214, an open end 216 and a closed end 218. The wall 206 may be rectangular in cross-section (as shown), circular, oval, pentagonal, hexagonal, or any other suitable shape or combination of shapes. The portion of the inner surface 208 with the apertures 212 may have a shape corresponding to the shape of the associated portion of the electrode array 106, or other electrode array that will be protected with the cover assembly 200, to facilitate the formation of a seal as is described below with reference to FIGS. 11-14. Similarly, the size and spacing of the apertures 212 should correspond to the size and spacing of the contacts of the electrode array that will be protected. The cover 202 is stiffer than the electrode array 106 and suitable materials for the cover include, but are not limited to, PTFE, PEEK and/or other materials that have low thermal and chemical reactivity.

The exemplary resilient insert 204 is configured to press the electrode array 106, or other electrode array, against the inner surface 208 of the apertured portion of the wall 206 in such a manner that a fluidic seal is formed between the array body and the apertured portion of the wall, as is described below with reference to FIGS. 11-14. The cross-sectional size and shape of the resilient insert 204 is such that the resilient insert will be compressed when both the electrode array 106 and the resilient insert are positioned within the cover 202. The cross-sectional size is largest at tip end in the illustrated embodiment so as to correspond to the smaller tip end of the associated electrode array. The compressed resilient insert 204 prevents movement of the electrode array 106 within the cover 202, especially at the tip end 124 where the flexible body is smaller, thereby maintaining the electrode array 106 in a straightened state. Suitable materials for the resilient insert 204 include, but are not limited to, silicone rubbers such as Silastic^{™} silicone rubber, polydimethylsiloxane (PDMS) elastomers, and thermoplastic elastomers (TPEs).

The electrode array 106 and the resilient insert 204 may be inserted through the open end 216 of the cover 202 and into interior volume 214 either simultaneously, as shown in FIG. 10, or one at a time. A removable stylet 220 may be used to drive the resilient insert 204 into the cover 202. The stylet 220 may be removed after the electrode array 106 and resilient insert 204 are positioned within the cover 202. The electrode array 106 (with or without the resilient insert 204) may be pulled out of cover 202 at the time of insertion into the cochlea.

Referring to FIGS. 11-14, the respective configurations of the electrode array 106 and the cover assembly 200 are such that the contact windows 128 will be aligned with the apertures 212 when the depth marker 132 reaches the cover open end 216. To that end, the center to center distance Dw between adjacent contact windows 128 is equal to the center to center distance D_{A} between adjacent cover apertures 212. The contact windows 128 are also slightly smaller (in length and width) than the cover apertures 212. As a result, when the resilient insert 204 presses the electrode array 106 against the portion of the cover inner surface 208 with the apertures 212, a seal 222 is formed between the flexible body outer surface 126 and the cover inner surface 208. The seal 222 extends along the length of the array 106, and around each of the cover apertures 212, thereby allowing fluid to reach the windows 128 and contacts 122 for impedance testing while preventing fluid from entering the cover 202. There is also a seal 224 formed around the perimeter of the cover inner surface 208 at the cover open end 216 between the cover inner surface and the outer surfaces 126 and 226 of the array flexible body 120 and the resilient insert 204.

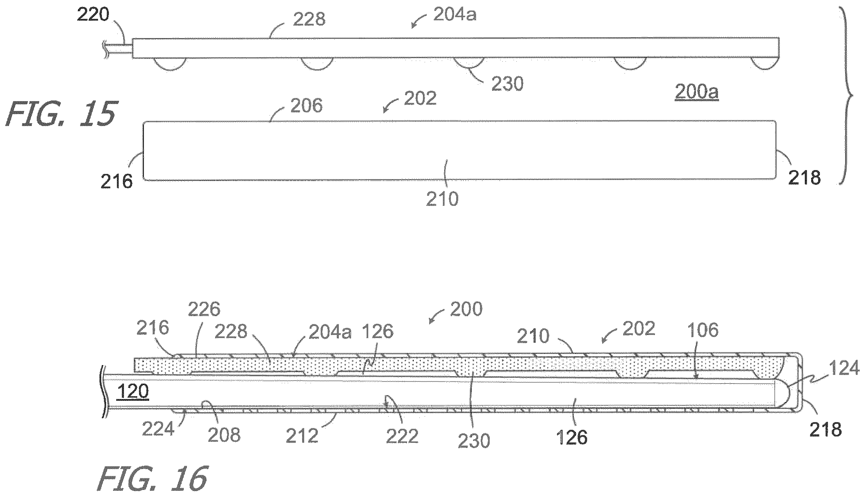

Another exemplary cover assembly is generally represented by reference numeral 200a in FIGS. 15 and 16. The cover assembly 200a is substantially similar to cover assembly 200 and similar elements are represented by similar reference numerals. For example, the cover assembly 200a includes the above-described cover 200 as well as a resilient member. Here, however, the resilient member 204a includes a main body 228 and a plurality of the protuberances 230. The protuberances 230, and associated portions of the main body 228, are compressed when the resilient member 204a and the electrode array 106 are located within the cover 202. As a result, the resilient insert 204a will presses the electrode array 106 against the portion of the cover inner surface 208 with the apertures 212, thereby forming a seal 222 is formed between the flexibly body outer surface 126 and the cover inner surface 208 in the manner described above. A seal 224 is also formed around the perimeter of the cover inner surface 208 at the cover open end 216 in the manner described above. The resilient insert 204a also maintains the electrode array in the straightened state.

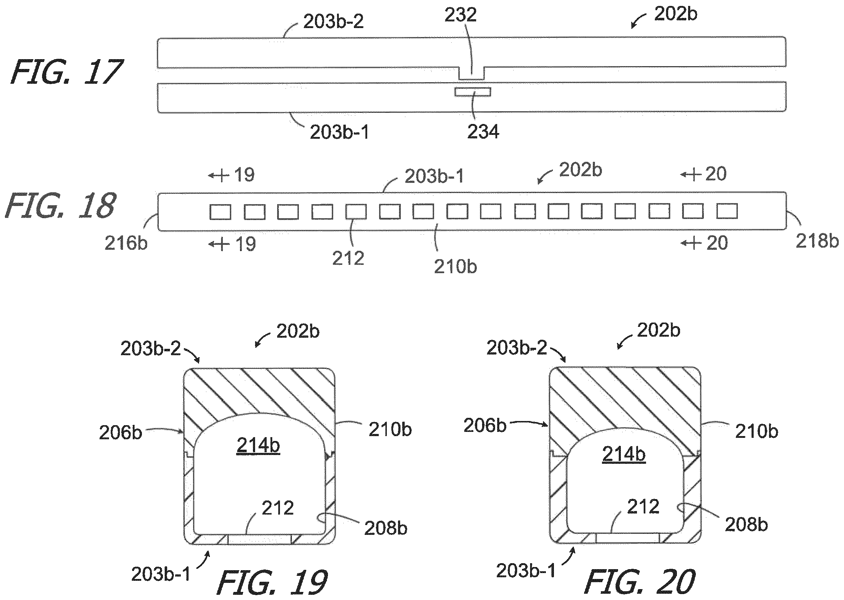

Turning to FIGS. 17-20, the exemplary electrode array cover 202b is configured so as to function in a manner similar to the cover assemblies 200 and 200a without the use of a resilient member. The cover 202b is a two-part structure that includes wall members 206b-1 and 206b-2 that may secured to one another with a latch 232 and recess 234, or any other suitable instrumentality, to form a wall 206b. The wall 206b of assembled cover 202b has an inner surface 208b formed by the inner surfaces of the first and second wall members 206b-1 and 206b-2, an outer surface 210b formed by the outer surfaces of the first and second wall members, and a plurality of spaced apertures 212 that extend through the first wall member from the inner surface the outer surface. The assembled cover 202b also includes an interior volume 214b, an open end 216b and a closed end 218b.

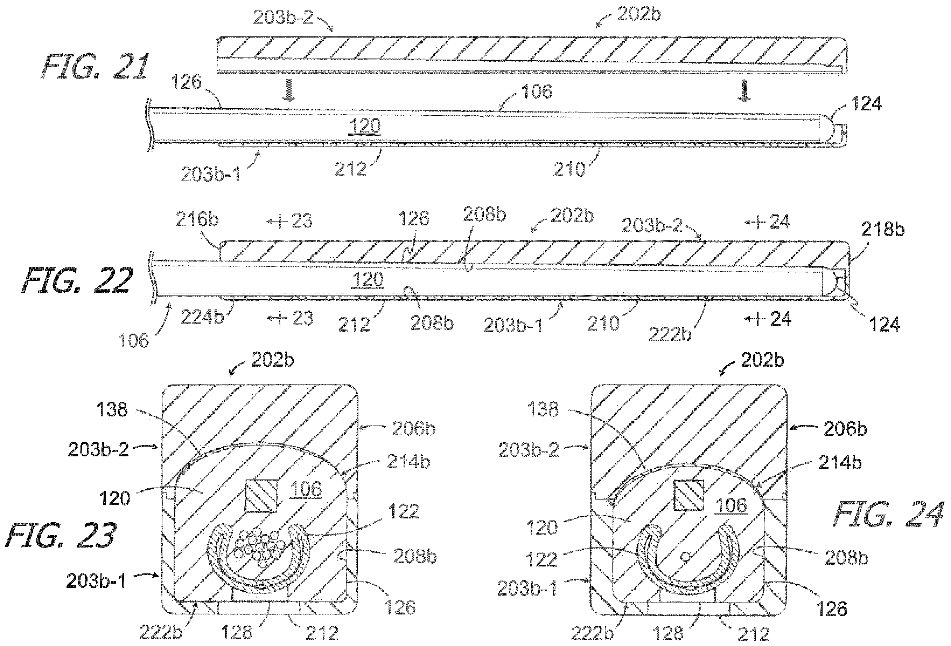

The cross-sectional size and shape of the inner surface 208b corresponds to the cross-sectional size and shape of the electrode array 106, or other electrode array that will be protected with the cover assembly 200b, to maintain the array in a straight state and to facilitate the formation of a seal as is described below with reference to FIGS. 21 and 22. To that end, the thickness of the second wall member 206b-1 increases from the cover open end 216b to the closed end 218b. The increase in thickness causes the interior volume 214b to taper in a manner corresponding to the taper of the electrode array 106. Similarly, the size and spacing of the apertures 212 should correspond to the size and spacing of the contacts of the associated electrode array in the manner described above.

As illustrated for example in FIGS. 21-24, the exemplary cover 202b is configured to press the electrode array 106, or other electrode array, against the inner surface 208b of the apertured portion of the wall 206b in such a manner that a fluidic seal is formed between the array body and the apertured portion of the wall, as is described above. The cross-sectional size and shape of the inner surface 208b (and interior volume 214b) along the length of the cover 202b are such that the array 106 will be compressed between the wall members 203b-1 and 203b-2, when the wall members are secured to one another. The second wall member 203b-2 also holds the electrode array body 120 against the first wall member 203-1. As a result, the electrode array 106 is kept straight and a seal 222b is formed between the flexibly body outer surface 126 and the cover inner surface 208b. The seal 222b extends along the length of the array 106, and around each of the cover apertures 212, thereby allowing fluid to reach the array windows 128 (FIGS. 23 and 24) and contacts 122 for impedance testing while preventing fluid from entering the cover 202b. There is also a seal 224b formed around the perimeter of the cover inner surface 208b at the cover open end 216b between the cover inner surface and the outer surface 126 of the array flexible body 120. The electrode array 106 may be removed from the cover 202b at the time of implantation by simply separating the wall members 206b-1 and 206b-2.

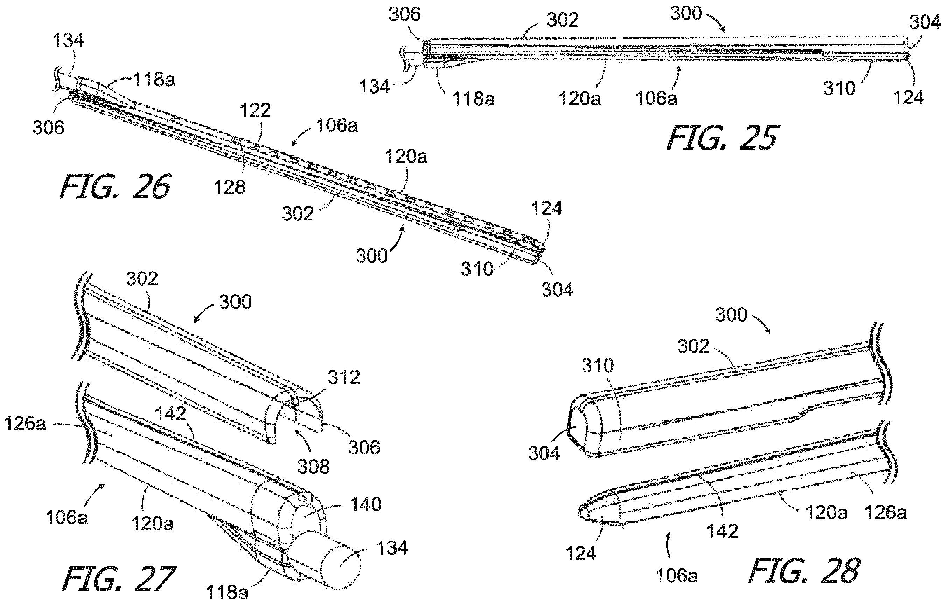

Braces may also be used to maintain an electrode array of a cochlear implant in a straightened state. Referring to FIGS. 25-28, a brace 300 may be used to maintain the electrode array 106a of a cochlear implant, such as a cochlear implant that includes a stimulation assembly 102 (FIG. 3) in a straightened state. The exemplary brace 300 may include a curved wall 302 (e.g., a U-shaped wall or a C-shaped wall), a tip end 304 and a rear end 306. The respective lengths of the brace 300 and the electrode array 106a may be such that, when the brace 300 is in the use position illustrated in FIGS. 25 and 26, the brace tip end 304 is adjacent to the electrode flexible body tip end 124 and the brace rear end 306 is adjacent to the rear end 140 of the wing 118a. The curved wall 302 defines an open region 308 for the electrode array 106a and also extends partially around the outer surface 126a of the flexible body 120a. The portion of the outer surface 126a with the openings 128 for the contacts 122 is not covered by the curved wall 302. The curved wall 302 may also include an enlarged area 310 adjacent to the tip end 304.

The brace 300 is stiffer than the electrode array 106a and, accordingly, the brace will maintain the electrode array 106a in a straightened state, which includes maintaining the tip end 124 in its unstressed molded shape that is a slightly curved in the direction of the contacts 122 with the enlarged area 310. The brace 300 will also any cover drug coatings (not shown here) that are located on the electrode array (see FIGS. 5 and 6). Suitable materials for the brace include, but are not limited to, thermoplastic materials such as PEEK, acrylic, ABS, and/or other materials that have low thermal and chemical reactivity.

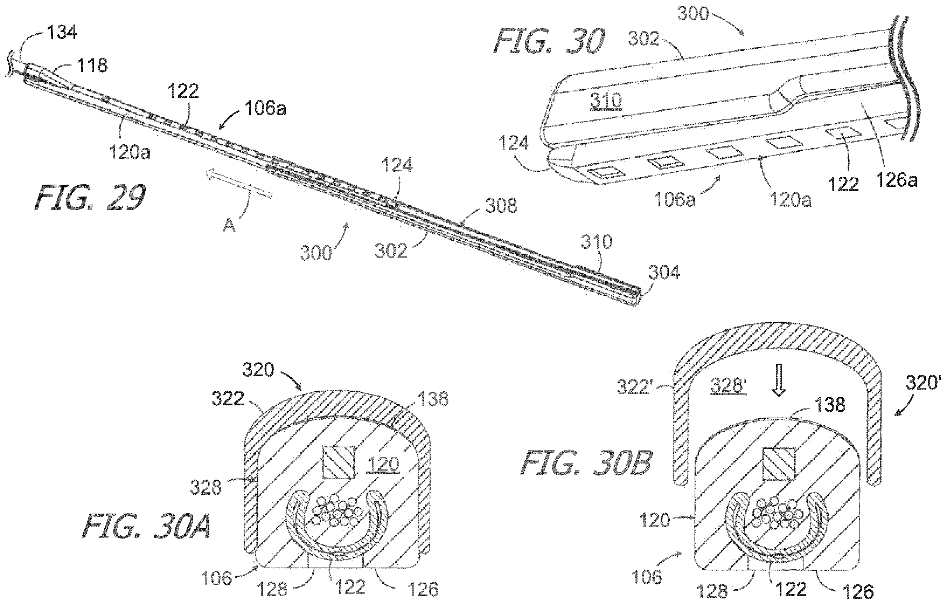

The brace 300 may be secured to the electrode array 106a in any suitable fashion. Referring to FIGS. 27 and 28, the electrode array 106a and the exemplary brace 300 are configured to interlock with one another. The flexible body 120a of the electrode array 106a, which is otherwise identical to electrode array 106, includes a longitudinally extending slot 142 with a narrow portion at the outer surface of the electrode body and a wide portion below the surface. The slot 142 extends from the tip end 124 of the flexible body 120a to the rear end 140 of the wing 118a. The brace 300 includes a corresponding longitudinally extending rail 312, with a wide portion and a narrow portion that are of the same or similar size and shape of the slot 142, that extends from the rear end 306 to a point adjacent to the tip end 304 and that is configured to fit into the slot 142. The brace 300 may be secured to the electrode array 106a by positioning the brace rear end 306 adjacent to the flexible body tip end 124 and inserting the rail 312 into the slot 142. Turning to FIG. 29, the brace 300 may then be moved over the flexible body 120a and the wing 118a of the electrode array 106a in the direction indicated by arrow A until the brace 300 reaches the position illustrated in FIGS. 25 and 26. The brace 300 may be removed from the electrode array 106a by sliding the brace off the electrode array in the opposite direction.

As illustrated for example in FIG. 30, the electrode array 106a and the brace 300 are configured in such a manner that a fluidic seal is formed between the outer surface 126a of the array body 120a and the inner surface of the curved wall 302. In particular, the slot 142 and rail 312 hold the inner surface of the curved wall 302 against the outer surface 126a of the array body 120a.

Braces similar to brace 300 may also be secured to electrode arrays without the above-described slot and rail arrangement. By way of example, but not limitation, shrinkable braces may be mounted onto electrode arrays. To that end, the brace 320 illustrated in FIG. 30A is substantially similar to brace 300 and similar elements are represented by similar reference numerals. The brace 320 includes a curved wall 322 and an open region 328 for the array 106. In the shrunken (or "cured") state illustrated in FIG. 30A, the open region 328 is slightly smaller in cross-section than the array body 120 and the curved wall 122 compresses slightly into the array body 120, thereby securing the brace 320 to the electrode array 106. The brace 320 is stiffer than the electrode array 106 and, accordingly, the brace will maintain the electrode array in a straightened state. A seal is also formed between the associate portion of the outer surface 126 and the inner surface of the brace 320 that prevents fluid ingress between the electrode array 106 and the brace.

Turning to FIG. 30B, the curved wall 322' of the pre-shrunk brace 320' defines an open region 328' that is slightly larger in cross-section than the array body 120 and the curved wall 322, which facilitates placement of the pre-shrunk brace 320' onto the array body 120.

Suitable shrinkable materials for the for the curved wall 322 include materials that shrink in response to the application of an external stimulus such as heat, cold, wetting or the removal of a force. By way of example, but not limitation, one suitable cold-shrink material is Ethylene Propylene Diene Monomer (EPDM) rubber, one suitable heat-shrink material is Fluorinated Ethylene Propylene (FEP) rubber. A cold temperature that activates a cold-shrink material may be a temperature that is between room temperature (about 21°C) and -40°C and, accordingly, cold shrink braces are held in the pre-shrunk state on a core that prevents shrinkage and remains in place until the pre-shrunk brace 320' is placed onto an electrode array. A hot temperature that activates a heat-shrink material may be a temperature that greater than 60°C. Such materials also allow the brace 320 to be peeled off of the electrode array 120 at the time of the cochlear implant surgical procedure.

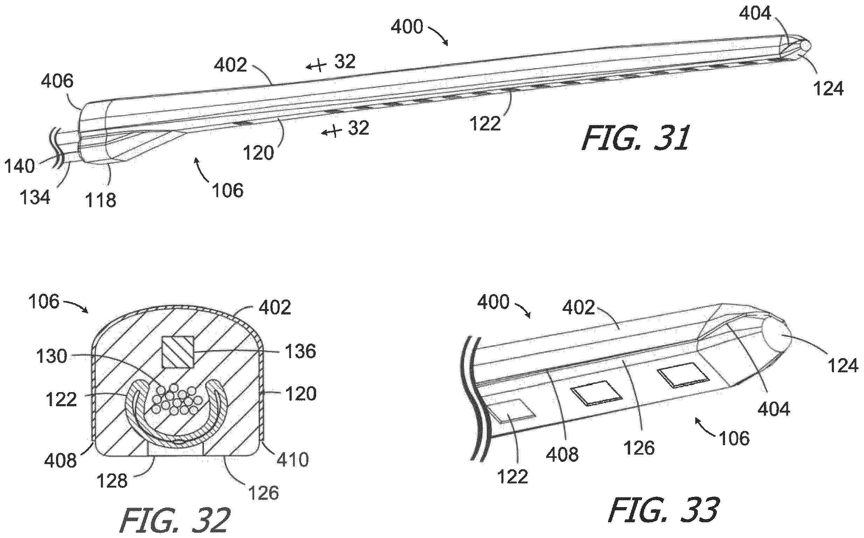

Another exemplary brace that may be attached to an electrode array of a cochlear implant, such as the exemplary electrode array 106 of cochlear implant 100, is generally represented by reference numeral 400 in FIGS. 31 and 32. Here, the brace 400 is in the form of a strip of pliable material 402 that includes a tip end 404, a rear end 406, and side edges 408 and 410. The brace 400 extends from the tip end 124 of the flexible body 120 to the rear end 140 of the wing 118, and around the outer surface 126 with the exception of the portion of the outer surface that includes the apertures 128. The brace 400 is stiffer than the electrode array 106 and, accordingly, the brace will maintain the electrode array 106 in a straightened state.

The strip 402, which is initially flat, is wrapped around the electrode array 106 in the manner illustrated in FIG. 31 to form the brace 400. The strip 402 may be formed from material that has an affinity to the material that forms the flexible body 120, i.e., the two materials will stick to one another without use of a separate adhesive, so that the brace 400 will stick to the electrode array 106 during shipping and storage, but will still allow the brace 400 to be peeled off. The strip 402 may, for example, be formed from silicone in those instances where the flexible body 120 is formed from silicone. Here, isopropyl alcohol (IPA) may be applied to the outer surface 126 of the flexible body 120 prior to the application of the strip 402. The IPA facilitates the elimination of creases and bubbles as the strip 402 is pressed onto the outer surface 126 and then quickly evaporates.

The brace 400 also fits tightly against the outer surface 126 of the array body 120, including at the tip end 404, rear end 406, and side edges 408 and 410, thereby forming a fluidic seal between the outer surface 126 and the inner surface of the strip of material 402 as shown in FIG. 33.

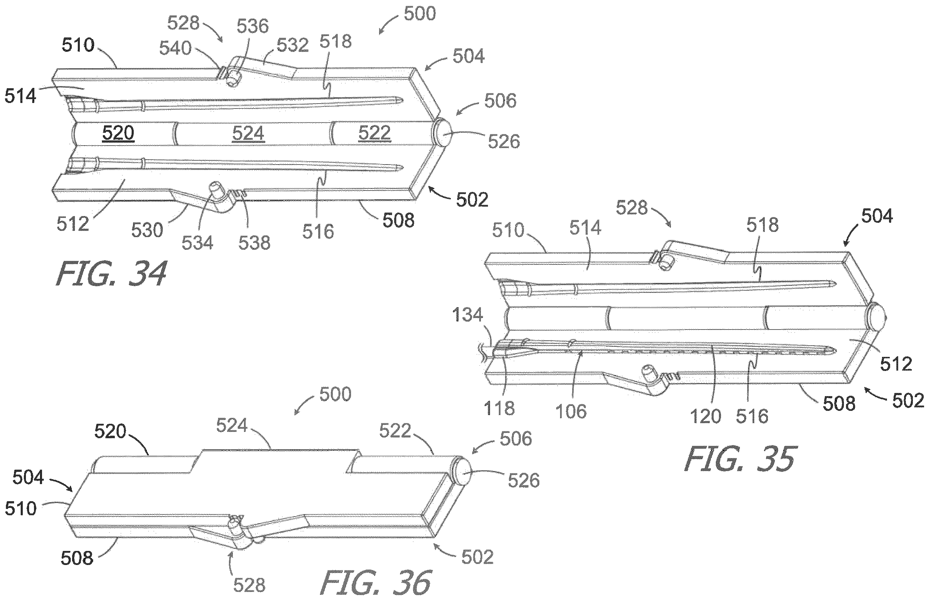

Cases may also be used to maintain an electrode array of a cochlear implant, such as the electrode array 106 of cochlear implant 100 (FIG. 3), in a straightened state and to protect the electrode array from moisture. One example of such a case is generally represented by reference numeral 500 in FIGS. 34-36. The exemplary case 500 includes a base 502 and a cover 504 that are pivotably secured to one another with a hinge 506 so that the case can be moved to and from the open state illustrated in FIGS. 34 and 35 and the closed state illustrated in FIG. 36. The base 502 and cover 504 may essentially identical (as shown) or may be different in one or more respects. The base 502 and cover 504 respectively include hard exterior portions 508 and 510 and resilient interior portions 512 and 514 with recesses 516 and 518. The recesses 516 and 518 have sizes and shapes that correspond to the size and shape of a respective one-half of the electrode array 106. The recesses 516 and 518 together form a storage volume, having the same size and shape as the associated electrode array (here, electrode array 106), that firmly holds the electrode array and maintains the electrode array in the straightened state when the case 500 is closed. The resilient interior portions 512 and 514 also create a seal when the case 500 is closed to protect the electrode array 106 from moisture. Suitable materials for the hard exterior portions 508 and 510 include, but are not limited to, thermoplastic materials such as PEEK, acrylic, ABS, and/or other materials that have low thermal and chemical reactivity, and suitable materials for the resilient interior portions 512 and 514 include, but are not limited to, silicone rubbers such as Silastic^{™} silicone rubber, polydimethylsiloxane (PDMS) elastomers, and thermoplastic elastomers (TPEs).

Although the case 500 is not limited to any particular type of hinge, the exemplary hinge 506 includes knuckles 520 and 522 on the base 502, knuckle 524 on the cover 504 and a pin 526. The case 500 may also be provided with a fastener 528 that maintains the base 502 and cover 504 in the closed state illustrated in FIG. 36. By way of example, but not limitation, the exemplary fastener 528 includes base and cover flanges 530 and 532, base and cover posts 534 and 536 that project away from the flanges 530 and 532, and base and cover grooves 538 and 540 that are configured to receive the posts 534 and 536. The posts 534 and 536 abut the flanges 530 and 532 when the base 502 and cover 504 are in the closed positions, thereby creating an interference that maintains the case 500 in the closed state.

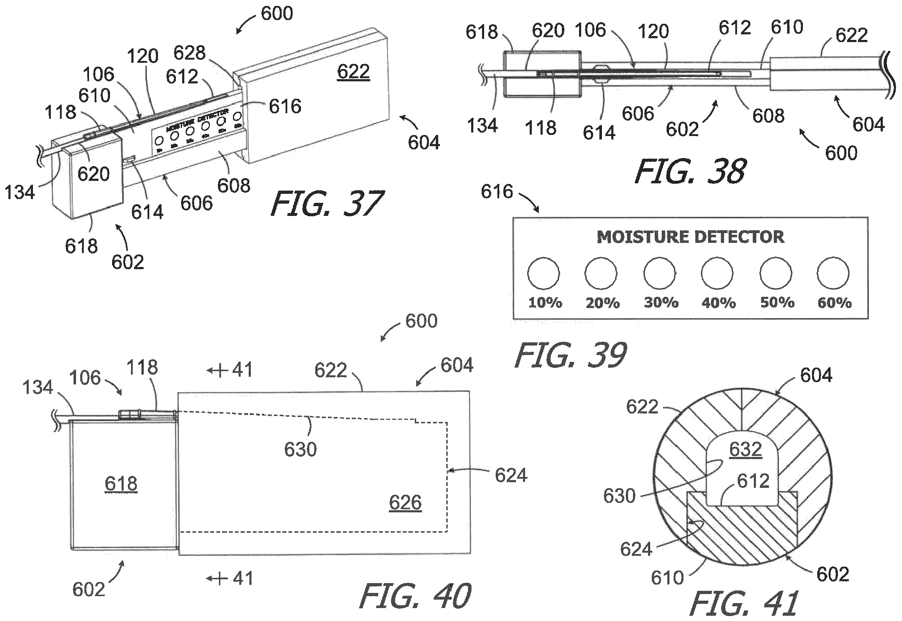

Another exemplary case that may be used to maintain an electrode array of a cochlear implant, such as the electrode array 106 of exemplary cochlear implant 100 (FIG. 3), in a straightened state and protect the electrode array from moisture is generally represented by reference numeral 600 in FIGS. 37-41. The exemplary case 600 includes a carrier 602 that is configured to support the electrode array 106 and an enclosure 604 into which portions of the carrier and electrode array may be inserted and removed.

The exemplary carrier 602 includes a T-shaped frame 606 with a base 608 and an array support 610 extending upwardly (in the illustrated orientation) from the base. The array support 610 includes a channel 612 on and in which the electrode array flexible body 120 is supported, a pair of latches 614 that enter recesses (not shown) within the enclosure 604 when the carrier is in the closed position (FIG. 40), and a moisture detector 616 (such as a cobalt dichloride-based moisture detector) that indicates the highest level of humidity that the electrode array is exposed to during shipping and storage. A handle 618, which abuts the enclosure 604 when the carrier 602 is in the closed position, is located at the end of the frame 606 and includes a channel 620 for the wing 118 and the tubular member 134. The channels 610 and 620 together form a continuous channel for the electrode array 106.

The exemplary enclosure 604 includes a plurality of outer walls 622 that together define an inner surface 624, an interior volume 626 for the carrier frame 606 and a portion of the electrode array 106 (here, the flexible body 120), and a frame entrance 628. The inner surface 624 includes an array contact surface 630 that combines with the channel 612 to define an electrode array storage volume 632. The cross-sectional size and shape of the storage volume 632 along its length corresponds to that of the flexible body 120. When the carrier 602 and electrode array 106 move from the open position illustrated in FIG. 37 to the closed position illustrated in FIG. 40, the corresponding portion of the electrode array 106 will be firmly held (and in some instances slightly compressed) between the carrier channel 612 and the enclosure array contact surface 630. As a result, the electrode array flexible body 120 is kept straight and a moisture-resistant seal is formed around the outer surface of the flexible body. Moisture ingress, if any, into the enclosure 604 will be detected by the moisture detector 616.

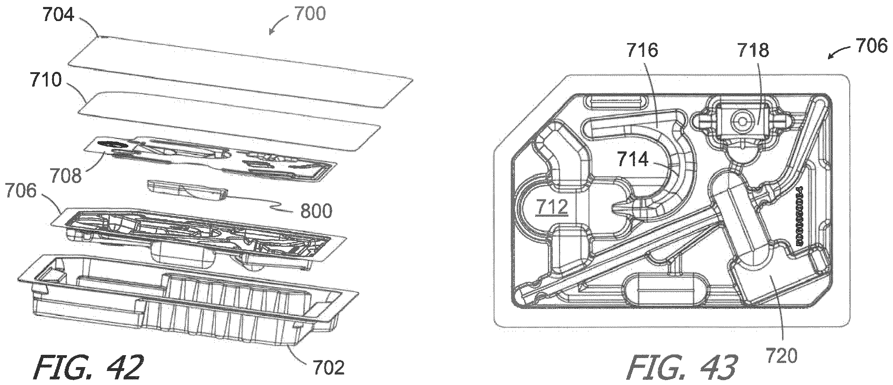

Electrode array cases may also be incorporated into the overall shipping and storage package for a cochlear implant (such as the cochlear implant 100 in FIG. 3), and one embodiment of such a package is generally represented by reference numeral 700 in FIG. 42. To that end, the exemplary packaging 700 includes an outer tray 702 that is covered by an outer lid 704 (e.g., a Tyvek^{®} lid), an inner tray 706 (with a tray top 708) that is covered by an inner lid 710 (e.g., a Tyvek^{®} lid). The inner tray 706 includes a compartment 712 for the cochlear implant stimulation assembly 102, a compartment 714 for the cochlear implant tubular member 134, a compartment 716 for the electrode array 106, a compartment 718 for a stylet, and a compartment 720 for an electrode array reloading tool. The packaging 700 also includes an electrode array case 800 that is sized and shaped such that it may be press fit into compartment 716 and may be used to maintain an electrode array, such as electrode array 106, in a straightened state and to protect the electrode array from moisture.

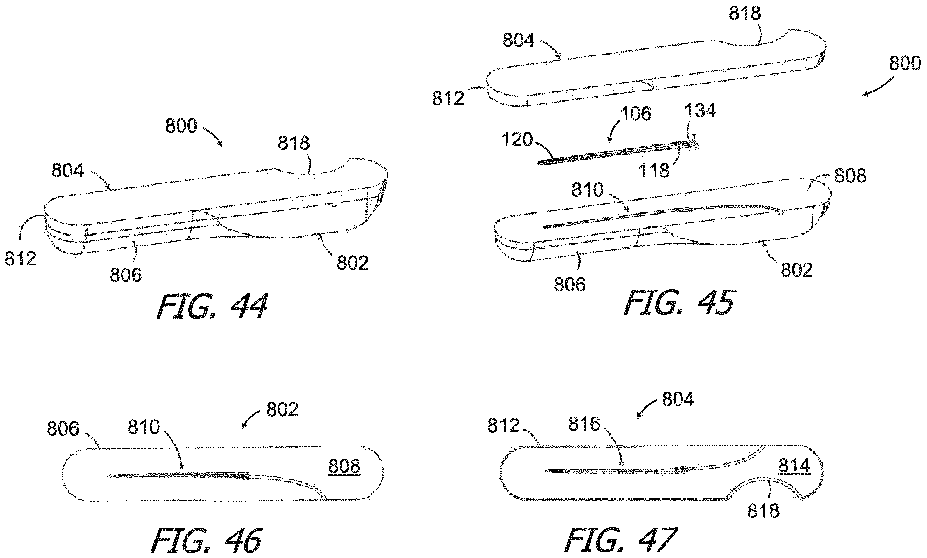

Turning to FIGS. 44-47, the exemplary case 800 includes a base 802 and a cover 804 that can be moved to and from the closed state illustrated in FIG. 44 and the open state illustrated in FIG. 45. The base 802 includes an outer surface 806, with a size and shape that corresponds to the size and shape of the associated portion of the compartment 716, and a top surface 808 with a recess 810. The cover 804 includes an outer surface 812, with a size and shape that corresponds to the size and shape of the associated portion of the compartment 716, and a bottom surface 814 with a recess 816. The recesses 810 and 816 together form a storage volume, having the same size and shape as the associated electrode array (here, electrode array 106 of the cochlear implant 100) and portion of the associated tubular member (here, tubular member 134), that firmly holds the electrode array and maintains the electrode array in the straightened state when the case 800 is closed and creates a tight fit (and seal) between the electrode array and the surfaces of the recesses 810 and 816 that prevents moisture ingress.

The base 802 and cover 804 may be formed from materials such as, for example, silicone rubbers such as Silastic^{™} silicone rubber, polydimethylsiloxane (PDMS) elastomers, and thermoplastic elastomers (TPEs). Such materials are self-adhesive and hydrophobic, which also contributes to formation of fluidic seal. In some instances, in order to facilitate separation of the cover 804 from the base 802 and removal of the cover from the tray compartment 716, the cover may include a recess 818 for a finger or tool.

Although the inventions disclosed herein have been described in terms of the preferred embodiments above, numerous modifications and/or additions to the above-described preferred embodiments would be readily apparent to one skilled in the art. The inventions also include any combination of the elements from the various species and embodiments disclosed in the specification that are not already described. It is intended that the scope of the present inventions extend to all such modifications and/or additions and that the scope of the present inventions is limited solely by the claims set forth below.

The invention relates to the following aspects:
1. An apparatus, comprising:
   a cochlear implant including an electrode array with an array body that defines an outer surface and a plurality of electrically conductive contacts on the array body; and
   an electrode array cover assembly including
      an electrode array cover including a wall, defining an inner surface and an outer surface, and a plurality of spaced cover apertures that extend through an apertured portion of the wall from the inner surface to the outer surface and that are at least substantially aligned with the electrically conductive contacts, and
      means, at least partially within the electrode array cover, for holding the array body straight and against the inner surface of the apertured portion of the wall in such a manner that a fluidic seal is formed between the array body and the apertured portion of the wall.
2. An apparatus as claimed in claim 1, wherein
   the contacts are embedded within the array body; and
   the array body includes a plurality of windows that respectively expose portions of the contacts.
3. An apparatus as claimed in claim 1 or claim 2, wherein
   the electrode array defines an apical end and a basal end;
   the assembly further comprises means for creating a fluidic seal adjacent to the basal end of the electrode array.
4. An apparatus as claimed in any one of claims 1 to 3, wherein
   the electrode array cover defines a longitudinal axis; and
   the wall defines a shape in a plane perpendicular to the longitudinal axis selected from the group consisting of rectangular, circular, oval, pentagonal, hexagonal, and combinations thereof.
5. An apparatus as claimed in any one of claims 1 to 4, wherein
   the electrode array defines a longitudinal axis and a perimeter that extends around the longitudinal axis; and
   the electrode array includes drug eluting material that is perimetrically offset from the contacts.
6. An apparatus as claimed in any one of claims 1a to 5a, wherein
   the electrode array body comprises a pre-curved electrode array body; and
   the electrode array includes a stiffener configured to maintain the electrode array body in a straightened state.
7. An apparatus as claimed in any one of claims 1 to 6, wherein
   the electrode array cover includes first and second separable cover members.
8. An apparatus, comprising:
   a cochlear lead including an electrode array with an array body that defines an outer surface and a plurality of electrically conductive contacts on the array body; and
   an electrode array cover assembly including
      an electrode array cover including a wall, defining an inner surface and an outer surface, and a plurality of spaced cover apertures that extend through an apertured portion of the wall from the inner surface to the outer surface and that are at least substantially aligned with the electrically conductive contacts, and
      a resilient insert at least partially within the electrode array cover and compressed between the inner surface of the electrode array cover and the outer surface of the electrode array;
   wherein the respective configurations of the array body and the resilient insert are such that the compressed resilient insert holds the array body straight and against the inner surface of the apertured portion of the wall in such a manner that a fluidic seal is formed between the array body and the apertured portion of the wall.
   a manner that a fluidic seal is formed between the array body and the apertured portion of the wall.
9. An apparatus as claimed in claim 8, wherein
   the contacts are embedded within the array body; and
   the array body includes a plurality of windows that respectively expose portions of the contacts.
10. An apparatus as claimed in claim 8 or claim 9, wherein
   the electrode array defines an apical end and a basal end;
   the respective configurations of the electrode array cover, the array body and the resilient insert are such a fluidic seal is formed adjacent to the basal end of the electrode array.
11. An apparatus as claimed in any one of claims 8 to 10, wherein
   the electrode array cover defines a longitudinal axis; and
   the wall defines a shape in a plane perpendicular to the longitudinal axis selected from the group consisting of rectangular, circular, oval, pentagonal, hexagonal, and combinations thereof.
12. An apparatus as claimed in any one of claims 8 to 11, wherein
   the electrode array defines a longitudinal axis and a perimeter that extends around the longitudinal axis; and
   the electrode array includes drug eluting material that is perimetrically offset from the contacts.
13. An apparatus as claimed in any one of claims 8 to 12, wherein
   the electrode array body comprises a pre-curved electrode array body; and
   the electrode array includes a stiffener configured to maintain the electrode array body in a straightened state.
14. An apparatus as claimed in any one of claims 8 to 13, wherein
   the resilient insert is formed from a shape memory polymer element or a shape memory alloy.
15. An apparatus as claimed in any one of claims 8 to 14, wherein
   the resilient insert includes a plurality of spaced projections that are in contact with the array body.
16. An apparatus as claimed in any one of claims 8 to 15, wherein
   the resilient insert defines an apical end, a basal end, a longitudinal axis and a cross-sectional area in a plane perpendicular to the longitudinal axis; and
   the cross-sectional area at the apical end is greater than the cross-sectional area at the basal end.
17. An apparatus, comprising:
   an electrode array cover including first and second separable wall members that are secured to one another, the first wall member defining an inner surface, an outer surface, and a plurality of spaced cover apertures that extend through an apertured portion of the first wall member from the inner surface to the outer surface, and the second wall member defining an inner surface and an outer surface;
   a cochlear lead including an electrode array with an array body that defines an outer surface and a plurality of electrically conductive contacts on the array body that are at least substantially aligned with the cover apertures; and
   wherein the respective configurations of the array body and the inner surface of the second wall member are such that the second wall member holds the array body against the inner surface of the apertured portion of the first wall member in such a manner that a fluidic seal is formed between the array body and the apertured portion of the first wall member.
18. An apparatus as claimed in claim 17, wherein
   the contacts are embedded within the array body; and
   the array body includes a plurality of windows that respectively expose portions of the contacts.
19. An apparatus as claimed in claim 17 or claim 18, wherein
   the electrode array defines an apical end and a basal end;
   the respective configurations of the first and second wall members and the array body are such a fluidic seal is formed at the basal end of the electrode array.
20. An apparatus as claimed in any one of claims 17 to 19, wherein
   the electrode array cover defines a longitudinal axis; and
   the wall defines a shape in a plane perpendicular to the longitudinal axis selected from the group consisting of rectangular, circular, oval, pentagonal, hexagonal, and combinations thereof.
21. An apparatus as claimed in any one of claims 17 to 20, wherein
   the electrode array defines a longitudinal axis and a perimeter that extends around the longitudinal axis; and
   the electrode array includes drug eluting material that is perimetrically offset from the contacts.
22. An apparatus as claimed in any one of claims 17 to 21, wherein
   the electrode array body comprises a pre-curved electrode array body; and
   the electrode array includes a stiffener configured to maintain the electrode array body in a straightened state.
23. An apparatus as claimed in any one of claims 17 to 22, wherein
   the electrode array defines a longitudinal axis and a perimeter that extends around the longitudinal axis; and
   the inner surface of the second wall member includes a longitudinally extending surface that faces the apertured portion of the first wall member and is in contact with a portion of the array body that is perimetrically offset from the contacts.
24. An apparatus as claimed in any one of claims 17 to 23, wherein
   the first and second separable wall members are secured to one another with a latch.
25. An apparatus, comprising:
   a cochlear implant including an electrode array with an array body that defines an outer surface and a plurality of electrically conductive contacts on the array body; and
   a brace secured to the outer surface of the array body and configured to maintain the array body in straight state.
26. An apparatus as claimed in claim 25, wherein
   the brace includes a curved wall that defines an open region in which the array body is located
27. An apparatus as claimed in claim 25 or claim 26, wherein
   the electrode array and the brace define respective stiffnesses; and
   the brace is stiffer than the electrode array.
28. An apparatus as claimed in any one of claims 25 to 27, wherein
   the electrode array and the brace are configured to interlock with one another.
29. An apparatus as claimed in claim 28, wherein
   the array body includes a longitudinally extending slot; and
   the brace includes a longitudinally extending rail that is configured to fit into the longitudinally extending slot.
30. An apparatus as claimed in claim 29, wherein
   the array body longitudinally extending slot includes a wide portion and a narrow portion; and
   the brace longitudinally extending rail includes a wide portion and a narrow portion.
31. An apparatus as claimed in any one of claims 25 to 27, wherein
   the brace is formed from a material that shrinks in response to the application of an external stimulus.
32. An apparatus as claimed in claim 31, wherein
   the material is selected from the group consisting of heat-shrink material and cold-shrink material.
33. An apparatus as claimed in any one of claims 25 to 27, wherein
   the brace is formed from a sheet of brace material that is wrapped at least partially around the array body.
34. An apparatus as claimed in claim 33, wherein
   the array body is formed from an array body material; and
   the brace material sticks to the array body material without adhesive.
35. An apparatus as claimed in any one of claims 25 to 34, wherein
   the electrically conductive contacts are not covered by the brace.
36. An apparatus as claimed in any one of claims 25 to 35, wherein
   the brace defines an inner surface; and
   a fluidic seal is formed between the brace inner surface and the array body outer surface.
37. An apparatus, comprising:
   a cochlear implant including an electrode array with an array body that defines an outer surface and a plurality of electrically conductive contacts on the array body; and
   a case, in which at least the array body is located, that is configured to maintain the array body in straight state.
38. An apparatus as claimed in claim 37, wherein
   the case includes first and second members that are movable between an open state and a closed state; and
   in the closed state, the first and second members together define a volume having the same size and shape as the array body.
39. An apparatus as claimed in claim 37, wherein
   the array body has a size and a shape;
   the case includes a base and a cover that are pivotably connected to one another and are movable between an open state and a closed state;
   the base includes a hard exterior portion and a resilient interior portion with a base recess having a size and shape that corresponds to the size and shape of one-half of the array body;
   the cover includes a hard exterior portion and a resilient interior portion with a cover recess having a size and shape that corresponds to the size and shape of the other half of the array body; and
   the base recess and the cover recess together form a storage volume in which the array body is located.
40. An apparatus as claimed in claim 39, wherein
   the electrode array includes a wing, with a size and a shape, at one end of the array body; and
   the base recess has a size and shape that corresponds to the size and shape of one-half of the array body and one-half of the wing;
   the cover recess has a size and shape that corresponds to the size and shape of the other half of the array body and the other half of the wing; and
   the base recess and the cover recess together form a storage volume in which the array body and wing are located.
41. An apparatus as claimed in claim 39 or claim 40, wherein
   the case includes a latch that maintains the base and cover in a closed state.
42. An apparatus as claimed in claim 37, wherein
   the case includes an enclosure and a carrier that supports the electrode array; and
   the carrier is movable between an open position where the electrode array body is located outside the enclosure and a closed position where the electrode array body is located inside the enclosure.
43. An apparatus as claimed in claim 42, wherein
   the electrode array body defines a length; and
   the enclosure includes an electrode array contact surface that engages the electrode array body along the length of the electrode array body when the carrier is in the closed position.
44. An apparatus as claimed in claim 42 or claim 43, wherein
   the carrier includes a channel in which the electrode array body is located.
45. An apparatus as claimed in claim 43 or claim 44, wherein
   wherein the electrode array body is at least firmly held between the carrier and the electrode array contact surface when the carrier is in the closed position.
46. An apparatus as claimed in any one of claims 42 to 45, wherein
   the electrode array includes a wing at one end of the array body; and
   the carrier is configured to support the wing such that the wing is located outside the enclosure when the carrier is in the closed position.
47. An apparatus as claimed in claim 37, wherein
   the array body has a size and a shape;
   the case includes a resilient base with a base recess having a size and shape that corresponds to the size and shape of one-half of the array body;
   the case includes a resilient cover, that is separable from the resilient base, with a cover recess having a size and shape that corresponds to the size and shape of one-half of the array body; and
   the base recess and the cover recess together form a storage volume in which the array body is located.
48. An apparatus as claimed in claim 47, wherein
   the electrode array includes a wing, with a size and a shape, at one end of the array body; and
   the base recess has a size and shape that corresponds to the size and shape of one-half of the array body and one-half of the wing;
   the cover recess has a size and shape that corresponds to the size and shape of the other half of the array body and the other half of the wing; and
   the base recess and the cover recess together form a storage volume in which the array body and the wing are located.
49. An apparatus as claimed in claim 47 or claim 48, further comprising:
   a tray including a plurality of compartments;
   wherein the resilient base and the resilient cover are located within one of the compartments.

## Claims

1. An apparatus, comprising:
a cochlear implant (100) including an electrode array (106, 106a) with an array body (120, 120a) that defines an outer surface (126, 126a) and a plurality of electrically conductive contacts (122) on the array body (120, 120a); and
a brace (300, 320, 320', 400) secured to the outer surface (126, 126a) of the array body (120, 120a) and configured to maintain the array body (120, 120a) in straight state.

2. An apparatus as claimed in claim 1, wherein
the brace (300, 320, 320', 400) includes a curved wall (302, 322, 322') that defines an open region in which the array body is located.

3. An apparatus as claimed in claim 1 or claim 2, wherein
the electrode array (106, 106a) and the brace (300, 320, 320', 400) define respective stiffnesses; and
the brace (300, 320, 320', 400) is stiffer than the electrode array (106, 106a).

4. An apparatus as claimed in any one of claims 1 to 3, wherein
the electrode array (106a) and the brace (300) are configured to interlock with one another.

5. An apparatus as claimed in claim 4, wherein
the array body (120a) includes a longitudinally extending slot (142); and
the brace (300) includes a longitudinally extending rail (312) that is configured to fit into the longitudinally extending slot (142).

6. An apparatus as claimed in claim 5, wherein
the array body longitudinally extending slot (142) includes a wide portion and a narrow portion; and
the brace longitudinally extending rail (312) includes a wide portion and a narrow portion.

7. An apparatus as claimed in any one of claims 1 to 3, wherein
the brace (320, 320') is formed from a material that shrinks in response to the application of an external stimulus.

8. An apparatus as claimed in claim 7, wherein
the material is selected from the group consisting of heat-shrink material and cold-shrink material.

9. An apparatus as claimed in any one of claims 1 to 3, wherein
the brace (400) is formed from a strip of brace material (402) that is wrapped at least partially around the array body.

10. An apparatus as claimed in claim 9, wherein
the array body (120) is formed from an array body material; and
the brace material (402) sticks to the array body material without adhesive.

11. An apparatus as claimed in any one of claims 1 to 10, wherein
the electrically conductive contacts (122) are not covered by the brace (300, 320, 320', 400).

12. An apparatus as claimed in any one of claims 1 to 11, wherein
the brace (300, 320, 320', 400) defines an inner surface; and
a fluidic seal is formed between the brace inner surface and the array body outer surface (126, 126a).
